# EUROPÄISCHE PATENTSCHRIFT

(11) **EP 1 911 421 B1**
(45) Veröffentlichungstag und Bekanntmachung des Hinweises auf die Patenterteilung: **20.06.2012**
(21) Anmeldenummer: 06019756.3
(22) Anmeldetag: 21.09.2006
(51) Int. Cl.: A61F 2/46, A61B 5/107, A61B 19/00

(54) **Vorrichtung zur Bestimmung der Veränderung eines Objektes**
Device for determining the change of an object
Dispositif destinés à la détermination de la modification d'un objet

(43) Veröffentlichungstag der Anmeldung: 16.04.2008
(73) Patentinhaber: BrainLAB AG, 85622 Feldkirchen (DE)
(72) Erfinder: Tuma, Gregor, 81675 München (DE)
(74) Vertreter: Schwabe - Sandmair - Marx

(56) Entgegenhaltungen:
- EP-A- 1 563 810
- EP-A- 1 654 997
- US-A1- 2003 153 829
- US-B1- 6 692 447

## Beschreibung

Die vorliegende Erfindung betrifft eine Vorrichtung zur Bestimmung der Längenveränderung eines Femurs, der in einem Anfangszustand mit einem Beckenknochen verbunden war und nach Loslösung von diesem Beckenknochen bearbeitet wurde, bevor er wieder mit diesem Beckenknochen verbunden wird. Der Femur wird im folgenden auch erstes Objekt genannt. Der Beckenknochen wird im folgenden auch zweites Objekt genannt.

Wenn ein chirurgischer Eingriff im Bereich eines Gelenks, wie zum Beispiel an einer Hüfte, vorgenommen wird, um zum Beispiel einen Teil eines mit dem Gelenk verbundenen Knochens, wie zum Beispiel den Femurkopf, durch ein Implantat zu ersetzen, sollte sichergestellt werden, dass nach Einsetzen des Implantats und Wiederverbinden des ein Implantat tragenden Femurs mit dem Becken eine Ausrichtung des Femurs relativ zu dem Becken vorliegt, welche für den Patienten einen normalen Bewegungsablauf wie bei einem gesunden Hüftgelenk ermöglicht.

Um die relative Position zwischen Femur und Hüfte bestimmen zu können und so auch Längenveränderungen des Femurs nach dem Anbringen eines Implantats quantifizieren zu können, ist es bekannt, dass ein Referenzstern sowohl unmittelbar an dem Beckenknochen, als auch unmittelbar an dem Femur, zum Beispiel durch Einschrauben in den jeweiligen Knochen, befestigt wird.

Aus der US 2003/0153829 A1 ist bekannt, dass ein Referenzstern mittels eines Spannelements an einem Femur in einem möglichst fest stehenden Lageverhältnis angebracht wird, wobei die Position eines femoralen Trackingmarkers eine Position auf dem Femur definiert.

Aus der EP 1 654 997 A1 und der EP 1 563 810 A1 sind unmittelbar am Femur angebrachte und in diesen eingeschraubte Referenzsterne bekannt.

Es ist eine Aufgabe der vorliegenden Erfindung eine Vorrichtung vorzuschlagen, welche eine einfachere Messung der Längenveränderung eines Gelenkteiles ermöglichen.

Diese Aufgabe wird durch den Gegenstand des einzigen Anspruchs gelöst.

Um die Längenveränderung eines ersten Objektes (d.h. des Femurs), welcher in einem Anfangszustand mit einem zweiten Objekt (d.h. ein Beckenknochen), über ein Hüftgelenk verbunden ist, zu erfassen ist oder wird in dem Anfangszustand ein erstes Referenzsystem, wie zum Beispiel ein Referenzstern oder eine andere Anordnung aus zum Beispiel reflektierenden Markern, unmittelbar und direkt mit dem zweiten Objekt (dem Beckenknochen) verbunden und beispielsweise in diesen eingeschraubt. Die Verbindung des zweiten Objektes mit dem ersten Referenzsystem kann auch bereits vor Beginn des Verfahrens vorliegen. Ein zweites Referenzsystem, wie zum Beispiel ein Referenzstern, ist mit dem ersten Objekt verbunden, wobei das zweite Referenzsystem nicht unmittelbar an dem ersten Objekt angeordnet ist und mittels eines bevorzugt elastischen Bandes in der näheren Umgebung des ersten Objektes, also an einer das erste Objekt umgebenden Haut (Mantel), angebracht ist. Beispielsweise kann ein auf einem Band aufgesetzter Referenzstern mittels des Bandes, welches um einen Bereich des Oberschenkels gelegt wurde, um das Band so möglichst unbeweglich im Verhältnis zur Hautoberfläche zu halten, fixiert werden. Somit ist es erfindungsgemäß nicht erforderlich, einen Referenzstern zum Beispiel mittels eines Pins unmittelbar an dem Femur zu befestigen, so dass auf einen chirurgischen Eingriff an dieser Stelle verzichtet werden kann.

Es wird mindestens eine Referenzmarke an dem ersten Objekt (an dem Femur) vor dem Auseinandernehmen des Gelenks erfasst, wobei eine Landmarke oder ein markierter Bereich auf bekannte Art mit einem Pointer, an welchem Marker angebracht sind, angefahren werden kann, um diesen Punkt zu akquirieren, das heißt, die Position dieses Punktes im Raum über ein bekanntes Navigationssystem zu erfassen.

Anschließend kann die Verbindung zwischen den beiden Objekten gelöst werden, um das zweite und/oder erste Objekt, also zum Beispiel den Femur, zu bearbeiten und insbesondere durch einen Bearbeitungsschritt die Länge zu verändern, um zum Beispiel einen beschädigten Femurkopf abzuschneiden und ein Implantat aufzusetzen. Sollen die beiden Objekte, also zum Beispiel Femur(Kopf) mit aufgesetztem Implantat und Beckenknochen, wieder zusammengefügt werden oder das aufgesetzte Implantat zum Beispiel bezüglich Form oder Lage überprüft werden, so ist es wichtig, die Geometrie, also die Länge, des veränderten Objektes genau zu kennen, damit sich das wieder zusammengesetzte Gelenk möglichst nicht von einem gesunden Gelenk oder zum Beispiel dem relativen Lageverhältnis der beiden Gelenkteile zueinander vor dem Eingriff unterscheidet.

Es wird unter Verwendung des mit einem Band an dem ersten Objekt befestigten zweiten Referenzsystemes das erste Objekt in eine Position gebracht, welche etwa der Position im Anfangs- oder Ausgangszustand entspricht. Da jedoch die Position wahrscheinlich nicht exakt mit der Anfangsposition übereinstimmt, da das zweite Referenzsystem nicht unmittelbar und starr mit dem ersten Objekt verbunden ist, wird zur Feinpositionierung und somit zur genauen Ausrichtung des ersten Objektes relativ zum zweiten Objekt die Position des zuvor erfassten mindestens einen Referenzpunktes erneut erfasst, wobei wiederum, wie oben beschrieben, ein bekannter Pointer verwendet werden kann. Dabei ist es jedoch nicht erforderlich, dass der gleiche Referenzpunkt angefahren wird. Es kann auch ein anderer Referenzpunkt auf dem ersten Objekt angefahren werden, wenn dessen Position relativ zu dem ersten Referenzpunkt bekannt ist. Beispielsweise kann der zweite Referenzpunkt eine zweite Landmarke mit bevorzugt bekannter relativer Anordnung zur zuvor erfassten ersten Landmarke oder jeder andere Punkt mit bekanntem Abstand in Relation zum zuvor erfassten ersten Referenzpunkt sein.

Somit kann erfindungsgemäß durch ein nicht-invasives Verfahren die Knochen- oder Beinlänge und ein Versatz (Offset) eines Gelenkteils erfasst oder gemessen werden, was insbesondere bei Hüftoperationen (THA;Total Hip Arthroplasty) wichtig ist, um nach der Implantation eines künstlichen Hüftgelenkes oder Hüftgelenkteiles die gewünschte Ausrichtung oder Relativposition zwischen den beiden Gelenkteilen, also Beckenknochen mit Hüftgelenkpfanne und Femurkopf, zu finden.

Die Erfindung bezieht sich auf eine Vorrichtung gemäß Anspruch 1 zum Bestimmen der Längenveränderung eines ersten Objektes, das in einem Anfangszustand mit einem zweiten Objekt über ein Gelenk verbunden ist, wobei ein

erstes Referenzsystem unmittelbar und bevorzugt fest an dem zweiten Objekt befestigt ist, ein zweites Referenzsystem nicht unmittelbar mit dem ersten Objekt verbunden aber an diesem oder in dessen Nähe bevorzugt im Wesentlichen nicht oder nur mit kleineren Verschiebungsmöglichkeiten an dem ersten Objekt angebracht ist und ein Zeigerinstrument oder Pointer vorgesehen ist, mit welchem die Position mindestens eines charakteristischen Punktes erfasst werden kann. Weiterhin ist ein Navigationssystem vorgesehen, mit welchem die Positionen des ersten Referenzsystems, des zweiten Referenzsystems und/oder des Pointers erfasst werden können, um durch einen Vergleich der erfassten Positionen in einem Anfangszustand bei noch verbundenem oder zusammengesetztem Gelenk mit den erfassten Positionen nach dem Verändern des ersten und/oder zweiten Objektes zu bestimmen, ob zum Beispiel die Länge des zweiten Objektes verändert wurde, wobei auch quantitativ ermittelt werden kann, wie groß diese Längenveränderung ist, ob also zum Beispiel ein Knochen mit einem aufgesetzten Implantat die richtige Länge aufweist oder zu lang oder zu kurz ist.

Die Erfindung wird nachfolgend anhand eines Ausführungsbeispieles beschrieben. Es zeigen:
- Figur 1: eine schematische Darstellung eines Hüftgelenkes im Anfangszustand vor Aufbringen eines Implantats auf den Femur;
- Figur 2: das in Figur 1 gezeigte Hüftgelenk nach Lösen der Gelenkverbindung und Abnehmen des Femurkopfes; und
- Figur 3: das Hüftgelenk nach Aufsetzen eines Femurkopf-Implantats und Zusammensetzung der Gelenkkomponenten.

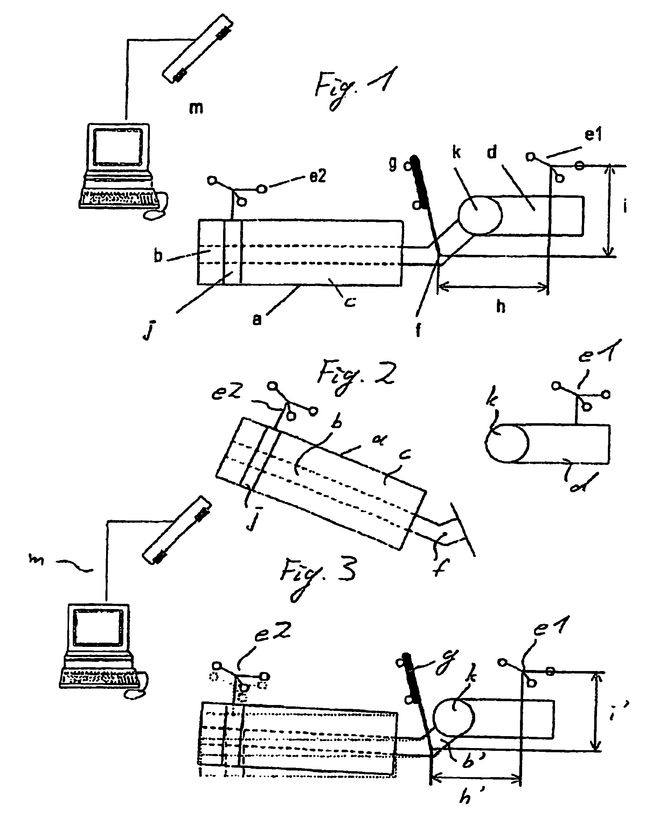

Figur 1 zeigt als Ausführungsform der Erfindung als längliches Objekt a einen Oberschenkel mit einem Femur oder Oberschenkelknochen als festen Kern b, um welchen als elastischer Mantel c Gewebe, wie zum Beispiel Haut und Muskeln, angeordnet sind. Das Objekt a ist durch den Kern b mittels eines Kugelgelenkes k mit einem zweiten Objekt d verbunden, an welchem zur Bestimmung oder Festlegung eines Koordinatensysterns ein Referenzstern oder Marker-Array e1 als erstes Referenzsystem, fest mit diesem zweiten Objekt d verbunden, vorgesehen ist. An dem Mantel c des ersten Objektes a ist mittels eines Bandes j um den Mantel c herum ein zweites Referenzsystem e2 vorgesehen, wobei sich der Mantel c und/oder das Band j relativ zu dem starren oder festen Kern b bewegen können, wenn zum Beispiel eine Bewegung des Objektes a vorliegt. Das Referenzsystem e1 des zweiten Objektes d bleibt jedoch fest oder starr mit dem zweiten Objekt d verbunden, selbst wenn dieses bewegt wird.

Mittels eines Pointers g, an welchem ebenso wie an den Referenzsystem e1 und e2 reflektierende Marker vorgesehen sind, wird ein Referenzpunkt f auf dem Kern b des ersten Objektes a erfasst, wobei die Positionen des ersten Referenzsystems e1, des zweiten Referenzsystems e2 und des Pointer g mittels eines bekannten Navigationssystems m, welches zum Beispiel Infrarotkameras aufweist, erfasst werden können. Ist die Position des Referenzsystemes e1 bekannt, so kann zum Beispiel nach einer bekannten Registrierung eines Bilddatensatzes (zum Beispiel CT) des zweiten Objektes d die Position des zweiten Objektes d im Raum bestimmt werden, welches beispielsweise ebenfso wie der Referenzstern e1 auch als Referenzsystem dienen kann.

In dem in Figur 1 gezeigten Ausgangszustand werden mittels des Navigationssystems m die Lage des zweiten Referenzsystems e2 relativ zum ersten Referenzsystem e1 und die Lage des mittels des Pointers g erfassten Referenzpunktes f relativ zum ersten Referenzsystem e1 erfasst und gespeichert, um einen Anfangszustand oder relative Lageverhältnisse zu definieren, welche nach Durchführung des chirurgischen Eingriffes wieder vorliegen sollten. Insbesondere kann zum Beispiel in Bezug auf das erste Referenzsystem e1 ermittelt werden in welcher Höhe i und in welcher Entfernung h der Referenzpunkt f liegt.

Figur 2 zeigt das in Figur 1 gezeigte Gelenk k nachdem die Gelenkverbindung zwischen dem ersten Objekt a und dem zweiten Objekt d gelöst wurde. Wie aus der schematischen Darstellung der Figur 2 ersichtlich, ist der zum Beispiel beschädigte Gelenkteil des ersten Objektes a, also der Femurkopf, vom Kern b oder Femur abgeschnitten worden und wird durch einen künstlichen Gelenkkopf b', also zum Beispiel eine Prothese, ersetzt.

Nachdem der künstliche Gelenkkopf b' auf den Kern b des ersten Objekts a aufgesetzt wurde, muss dieses relativ zum zweiten Objekt d richtig positioniert werden, um die (Kugel-)Gelenkverbindung in dem in Figur 1 gezeigten Ausgangszustand mit dem richtigen Lageverhältnis wieder herzustellen. Jedoch ist in diesem in Figur 3 gezeigten Zustand die Längenveränderung oder Veränderung der Geometrie und die genaue Position des ersten Objektes a zum Beispiel durch ein Verrutschen des Bandes j nicht genau bekannt.

Um die Veränderung der Geometrie nach dem Schneiden des Kerns b des ersten Objektes a zu messen, wird der zuvor erfasste Referenz- oder Markerpunkt f auf dem Kern b des ersten Objekts a verwendet. Dabei wird das mit dem ersten Objekt a verbundene zweite Referenzsystem e2 nur verwendet, um die relative Lage und Positionierung der beiden Objekte zueinander bezüglich der Rotationsfreiheitsgrade des Objektes a näherungsweise mit Hilfe des Navigationssystemes in der Art wiederzufinden, wie eine vom Anwender definierte Ausgangslage vor der Trennung der Objekte dem Navigationssystem als Referenzlage für spätere Wiederholungsmessungen zur Speicherung bekannt gemacht wurde, also um zum Beispiel eine Grobpositionierung des ersten Objektes a relativ zum zweiten Objekt d vorzunehmen. Somit kann aufgrund der möglichen Relativbewegung zwischen dem zweiten Referenzsystem e2 und dem ersten Objekt a zum Beispiel nur eine Ausrichtung oder Positionierung des ersten Objektes a bezüglich der drei Rotationsfreiheitsgrade durchgeführt werden.

Nachdem die beiden Objekte a und d an dem Kugelgelenk k wieder verbunden worden sind, werden die Drehfreiheitsgrade so angepasst, dass die ursprüngliche Orientierung innerhalb einer gewissen Toleranzgrenze gefunden werden kann. Diese Toleranzgrenze hängt von der Veränderung der relativen Lage des zweiten Referenzsystemes e2 relativ zum ersten Objekt a ab und beträgt zum Beispiel weniger als 3° für alle Drehfreiheitsgrade.

Insbesondere ist die Veränderung von zweien der drei Translationsfreiheitsgrade von Interesse, wobei die Veränderung der Relativposition der Referenzmarke f zum Beispiel in Bezug auf das erste Referenzsystem e1 ermittelt werden kann, um so zum Beispiel festzustellen, dass ein zu kurzes Implantat b' aufgesetzt wurde.

Nach einer erneuten Aufnahme des Refernenzpunktes f unter Beibehaltung einer im Rahmen der Toleranzgrenzen ähnlichen Ausrichtung zur Referenzlage des Objektes a können nun insbesondere die Veränderungen von 2 Distanzen, also zum Beispiel die Veränderung der vorher erfassten Höhe i und Entfernung h ermittelt werden. Dabei kann z.B. anhand der neu gemessenen Entfernung h' und Höhe i' festgestellt werden, dass ein zu kurzes Implantat eingesetzt wurde, oder die Implantatpositionierung innerhalb der Körperstruktur so ungünstig gewählt wurde, dass sich in einer Richtung durch die Veränderung dieses Freiheitsgrades zum Beispiel eine Verkürzung oder Verlängerung der Körperstruktur im Vergleich zur Aussgangsituation ergibt (im Rahmen der Hüftgelenksoperation oder Total Hip Arthroplasty (THA) wäre dies zum Beispiel die Beinlänge). Es kann weiterhin entlang der zweiten zur ersten Richtung senkrechten Distanz eine Veränderung ermittelt werden, die im Rahmen der THA zum Beispiel Einfluss auf die Bandspannung des Gelenkes hat, sowie die Hebelwirkung des Beines beeinflusst.

## Patentansprüche

1. Vorrichtung zum Bestimmen der Längenveränderung eines Femurs (b), der in einem Anfangszustand mit einem Beckenknochen (d) über ein Gelenk (k) verbunden ist, bestehend aus einem ersten Referenzsystem (e1), das unmittelbar und bevorzugt fest an dem Beckenknochen (d) befestigbar ist, einem zweiten Referenzsystem (e2) mit einem Band (j), das an einer den Femur (b) umgebenden relativ zum Femur (b) bewegbaren Haut (c) mittels des Bandes (j) um die Haut (c) herum, welches sich relativ zu dem Femur (b) bewegen kann, anbringbar ist und mit einem Zeigerinstrument oder Pointer (g), mit welchem die Position mindestens eines charakteristischen Punktes (f) auf den Femur (b) erfasst werden kann, und mit einem Navigationssystem (m), mit welchem die Positionen des ersten Referenzsystems (e1), des zweiten Referenzsystems (e2) und des Pointers (g) erfasst werden können, und die Längenveränderung des Femurs (b) bestimmt werden kann.

## Claims

1. A device for determining the change in length of a femur (b) which in an initial state is connected to a pelvic bone (d) via a joint (k), consisting of: a first reference system (e1) which can be fastened directly and preferably fixedly to the pelvic bone (d); a second reference system (e2) which comprises a belt (j) and can be attached to a skin (c), which surrounds the femur (b) and can be moved relative to the femur (b), around the skin (c) by means of the belt (j) which can be moved relative to the femur (b); and comprising a pointer instrument or pointer (g) using which the position of at least one characteristic point (f) on the femur (b) can be detected, and a navigation system (m) using which the positions of the first reference system (e1), the second reference system (e2) and the pointer (g) can be detected and the change in length of the femur (b) can be determined.

## Revendications

1. Dispositif pour déterminer la variation de longueur d'un fémur (b) qui, dans un état initial, est relié à un os de bassin (d) par l'intermédiaire d'une articulation (k), constitué d'un premier système de référence (e1) qui peut être fixé directement et de préférence solidement sur l'os de bassin (d), d'un second système de référence (e2) avec une bande (j), qui peut être fixé sur une peau (c) entourant le fémur (b) et mobile par rapport au fémur (b), autour de la peau (c) au moyen de la bande (j), laquelle bande peut se déplacer par rapport au fémur (b), et comportant un instrument indicateur ou un pointeur (g) au moyen duquel la position d'au moins un point caractéristique (f) sur le fémur (b) peut être détectée, et un système de navigation (m) qui permet de détecter les positions du premier système de référence (e1), du second système de référence (e2) et du pointeur (g), et de déterminer la variation de longueur du fémur (b).
